# EUROPÄISCHE PATENTSCHRIFT

(11) **EP 2 171 032 B1**
(45) Veröffentlichungstag und Bekanntmachung des Hinweises auf die Patenterteilung: **05.08.2015**
(21) Anmeldenummer: 08774382.9
(22) Anmeldetag: 26.06.2008
(51) Int. Cl.: C12M 1/00, C12M 1/02, C12M 1/06, C12M 1/107

(54) **FERMENTATIONSANLAGE**
FERMENTATION PLANT
INSTALLATION DE FERMENTATION

(30) Priorität: 26.06.2007 DE 202007009095 U
(43) Veröffentlichungstag der Anmeldung: 07.04.2010
(73) Patentinhaber: agratec Invest AG, 12489 Berlin (DE)
(72) Erfinder: POLZIN, Manfred, 17166 Gross Wokern (DE)
(74) Vertreter: Reininger, Jan Christian
(86) Internationale Anmeldenummer: PCT/EP2008/058208
(87) Internationale Veröffentlichungsnummer: WO 2009/000900

(56) Entgegenhaltungen:
- EP-A- 0 617 120
- WO-A-02/094978
- DE-A1- 2 029 585
- DE-A1- 2 210 735
- DE-A1- 3 239 304
- DE-A1-102005 057 978
- DE-T2- 60 025 178
- DE-U1-202004 017 610
- FR-A- 1 226 289
- US-A- 5 227 136
- US-A1- 2004 063 193

## Beschreibung

Die vorliegende Erfindung betrifft eine Fermentationsanlage für biologisches Material mit den Merkmalen des Oberbegriffs von Anspruch 1.

Im Sinne der vorliegenden Erfindung umfasst die Fermentation biologischen Materials alle aeroben und anaeroben Vorgänge, bei denen in einer Bioreaktion mittels Bakterien-, Pilz-, Zellkulturen oder durch den Zusatz von Enzymen biologisches Material umgesetzt wird.

Eine Fermentationsanlage umfasst beispielsweise einen Fermentationsbehälter zur Aufnahme biologischen Materials, wobei der Fermentationsbehälter entlang einer Behälter-Erstreckungsachse erstreckt ausgebildet ist und mindestens ein um eine Rührwerk-Drehachse rotierbar angeordnetes Rührwerk zum Rühren aufgenommenen biologischen Materials im Fermentationsbehälter. Das Rührwerk weist dabei mindestens eine Rührstrebe auf, die im Bereich der Rührwerk-Drehachse festgelegt ist und die Rührwerk-Drehachse ist im Wesentlichen senkrecht zur Behälter-Erstreckungsachse angeordnet. Derartige Fermentationsanlagen finden weite Anwendung beispielsweise bei der Herstellung von Biogas. Entlang der Erstreckungsrichtung des Fermentationsbehälters sind oftmals eine Mehrzahl, üblicherweise gleicher Rührwerke angeordnet. Dies ist zum Beispiel bei einem so genannten Propfenstromreaktor für die Herstellung von Biogas realisiert.

Eine solche Fermentationsanlage ist beispielsweise aus der EP 0 617 120 bekannt. Das in diesem Dokument beschriebene Rührwerk zeigt eine Mehrzahl geradliniger Rührstreben, die sich im Wesentlichen senkrecht von der Rührwerk-Drehachse weg erstrecken. Die Rührstreben gehen in ihren von der Rührwerk-Drehachse beabstandeten Endbereichen in eine Querstrebe über. Diese Querstrebe erstreckt sich im Wesentlichen parallel zur Rührwerk-Drehachse.

Für die Fermentation kommt biologisches Material insbesondere in Form von Monosubstraten, beispielsweise als Silagen, zur Anwendung. Derartige Silagen stellen aufgrund ihres hohen Trockensubstanz-Gehaltes, der zu 95% aus organischer Substanz besteht, besondere Anforderungen bei der Fermentation. Durch den hohen Anteil an organischer Substanz, die in einem Fermentationsprozess beispielsweise zu 80 % abgebaut wird, muss die Verfahrenstechnik der Fermentationsanlage an die veränderten Anforderungen angepasst werden. In Fermentationsanlagen, die mit Silagen beschickt werden, werden pro Tag höhere Mengen organischer Trockensubstanz eingebracht als z.B. in Anlagen, die mit Abfallmaterial, beispielsweise mit Gülle, beschickt werden. Man spricht daher davon, dass Fermentationsanlagen beim Einsatz von Silagen mit einer höheren "Raumbelastung" betrieben werden. Dadurch ergibt sich für derartige Anlagen eine Verkürzung der hydraulischen Verweilzeit des Substrates im Fermentationsbehälter.

Insbesondere bei der anaeroben Umsetzung von Silagen kann es durch den starken Abbau der organischen Bestandteile zu einer Verflüssigung und Verzuckerung des Silagen-Substrates kommen. Beim Einsatz von Fermentationsanlagen mit bekannten Rührwerken besteht insbesondere die Gefahr der Entmischung einzelner Substratbestandteile, was zur Verringerung der Gasausbeute führt. Außerdem kann es trotz der Bewegung der Rührwerke zu einer unzureichenden Wärme-, Feuchtigkeits-, Nährstoff- oder Bakterienverteilung im biologischen Material kommen, was den Gasertrag weiter reduziert.

Der vorliegenden Erfindung liegt somit die Aufgabe zu Grunde, eine Fermentationsanlage zu schaffen, die für die Fermentation biologischen Materials insbesondere in Form von Silagen eine verbesserte Durchmischung bietet.

Diese Aufgabe wird durch eine Fermentationsanlage mit den Merkmalen des Anspruchs 1 gelöst.

Erfindungsgemäß ist vorgesehen, dass die Rührstrebe ausgehend von einem ersten Befestigungsabschnitt im Bereich der Rührwerk-Drehachse über einen von der Rührwerk-Drehachse beabstandeten Umkehrbereich zurück in Richtung der Rührwerk-Drehachse verläuft.

Eine derart ausgestaltete Rührstrebe durchgreift das im Fermentationsbehälter befindliche biologische Material nicht nur auf dem Weg ausgehend von der Rührwerk-Drehachse hin zum Umkehrbereich sondern zusätzlich auch noch in weiteren Bereichen, die zwischen dem Umkehrbereich und der Rührwerk-Drehachse angeordnet sind. Dies ist vorteilhaft, weil eine bessere Durchmischung des biologischen Materials gewährleistet wird. Dadurch lässt sich eine homogene Mischung des biologischen Materials und seiner Abbauprodukte bei einer im Wesentlichen homogenen Temperaturverteilung erreichen. Eine weitere wichtige Funktion der Rührwerke besteht darin, das Aufsteigen von Gasbläschen durch die erzeugte Bewegung des biologischen Materials und seiner Abbauprodukte zu unterstützen.

Als Umkehrbereich im Sinne der vorliegenden Erfindung gilt ein von der Rührwerk-Drehachse beabstandeter Bereich, in der die Rührstrebe ihre Erstreckungsrichtung derart verändert, dass die veränderte Erstreckungsrichtung eine Ebene, in der die Rührwerk-Drehachse liegt, schneidet. Das Merkmal "zurück in Richtung der Rührwerk-Drehachse" ist daher nicht so eng auszulegen, dass der Verlauf der Rührstrebe vom Umkehrbereich ausgehend auf die eindimensionale Rührwerk-Drehachse ausgerichtet sein muss.

Im Zusammenhang mit sämtlichen Varianten der Fermentationsanlage ist offenbart, den Fermentationsbehälter als im Wesentlichen geschlossenen Behälter auszubilden. Dieser geschlossene Behälter umfasst jedoch zumindest eine Eintragsöffnung zum Einbringen beispielsweise von Impf- oder Frischgut und zumindest eine Austragsöffnung.

Eine bevorzugte Variante der Fermentationsanlage sieht vor, dass das Rührwerk bei einer Rotation um die Rührwerk-Drehachse ein Rührwerk-Volumen umschreibt und die Rührstrebe des Rührwerks zumindest abschnittsweise in Form von Rührstreben-Volumenabschnitten im Inneren des Rührwerk-Volumens verläuft und/oder das Rührwerk im Inneren des umschriebenen Rührwerk-Volumens mindestens eine sich bei der Rotation des Rührwerks mitdrehende Rührzusatzstrebe aufweist.

Als Rührwerk-Volumen wird hier der Raum verstanden, der bei einer vollständigen Drehung des Rührwerkes um die Rührwerk-Drehachse durch die mindestens eine an der Rührwerk-Drehachse festgelegte Rührstrebe, umschlossen wird. Die mindestens eine Rührstrebe lässt sich dabei zum einen derart anordnen, dass diese zusammen mit der Rührwerk-Drehachse in einer gemeinsamen Ebene liegen und somit ein ebenes Rührwerk ausbilden. Zum anderen lässt sich die mindestens eine Rührstrebe selbstverständlich in allen denkbaren gekrümmten Verläufen von der Rührwerk-Drehachse weg in einen Umkehrbereich führen, aus dem die Rührstrebe anschließend im Sinne der vorangehenden Definition in Richtung der Rührwerk-Drehachse verläuft. Bei dieser Variante werden dreidimensionale Rührwerke ausgebildet.

Dadurch dass Rührstreben-Volumenabschnitte und/oder Rührzusatzstreben vorgesehen sind, wird im Rührwerk-Volumen befindliches biologisches Material und dessen Abbauprodukte besser durchmischt und eine im Wesentlichen homogene Temperaturverteilung kann erzielt werden. Außerdem wird das Aufsteigen von Gasbläschen gefördert und somit die Gasausbeute bei der Biogasproduktion erhöht.

Unter einer im Wesentlichen gleichen Temperaturverteilung des biologischen Materials werden Temperaturunterschiede von wenigen Grad bevorzugt von unter einem Grad Celsius verstanden.

Im Sinne der vorliegenden Erfindung sind Rührstreben und/oder Rührzusatzstreben alle geradlinig und/oder gekrümmt erstreckten Bauelemente, die von der Rührwerk-Drehachse weg verlaufen. Diese Streben weisen beliebige hohle oder gefüllte Querschnittsprofile auf und können aus allen für den Zweck des Mischens biologischen Materials denkbaren Materialien gefertigt sein. Die Streben sind dabei einstückig oder modular aufgebaut, wobei sich in beiden Fällen mit dem Verlauf der jeweiligen Strebe deren Querschnittsprofil auch ändern kann.

Bevorzugt erstreckt sich die Rührzusatzstrebe zwischen zwei Rührstreben-Kontaktabschnitten, d.h. die Rührzusatzstrebe ist an zwei Bereichen der Rührzusatzstrebe befestigt, oder zwischen einem Rührstreben-Kontaktabschnitt und einem Drehachsen-Kontaktabschnitt im Bereich der Rührwerk-Drehachse, d.h. die Rührzusatzstrebe ist mit seinem einen Endabschnitt an der Rührwerk-Drehachse festgelegt und mit seinem anderen Endabschnitt an der Rührstrebe. Dabei sind zwangsläufig Verzweigungen zwischen Streben des Rührwerkes vorgesehen. Diese Verstrebungen sind bevorzugt so positioniert, dass eine verbesserte Durchmischung des biologischen Materials im Rührwerk-Volumen gewährleistet ist.

Bei der erfindungsgemäßen Ausführungsformen der Fermentationsanlage ist vorgesehen, dass die Rührstrebe und die Rührzusatzstrebe in einer Rührwerk-Ebene angeordnet sind, in der auch die Rührwerk-Drehachse verläuft. Dies stellt einen besonders einfachen Aufbau des Rührwerkes dar.

Diese Ausführungsformen sind derart ausgebildet, dass das Rührwerk eine erste Rührstrebe und eine zweite Rührstrebe aufweist, wobei die erste Rührstrebe in einer ersten Halbebene der Rührwerk-Ebene verläuft und die zweite Rührstrebe in einer durch die Rührwerk-Drehachse von der ersten Halbebene getrennten zweiten Halbebene der Rührwerk-Ebene verläuft. Das gesamte Rührwerk erstreckt sich dabei mit seinen Streben im Wesentlichen zweidimensional. Das Rührwerk-Volumen ist durch die Kombination des bei einer vollständigen Drehung durch die erste Rührstrebe und durch die zweite Rührstrebe umschlossenen Raumes definiert.

Der Fermentationsbehälter weist einen Behälterboden auf, der entlang der Rührwerk-Drehachse in Richtung des Behälterbodens geschnitten eine Querschnittskontur mit zwei parallel zueinander verlaufenden, im Wesentlichen senkrecht zur Rührwerk-Drehachse angeordneten Behälterseitenwänden aufweist, zwischen denen sich der Behälterboden mit einer den Fermentationsbehälter nach unten angrenzenden Bodenkontur erstreckt.

Die Fermentationsanlage sieht vor, dass der Behälterboden eben ausgebildet ist und eine im Wesentlichen rechtwinklig zu den Behälterseitenwänden verlaufende geradlinige Bodenkontur aufweist. Ein derartiger Fermentationsbehälter ist im Wesentlichen würfel- oder quaderförmig aufgebaut und lässt sich einfach errichten.

Bevorzugt ist im Behälterboden mindestens ein Abzugskanal vorgesehen, der sich entlang oder quer zur Behälter-Erstreckungsachse zwischen den Behälterseitenwänden erstreckt. Ein solcher Abzugskanal liegt noch tiefer als das Niveau des Behälterbodens und dient dem Sammeln und Abführen dünnflüssiger Abbauprodukte, die bei der Fermentation des biologischen Materials entstehen.

Unabhängig davon ob ein Abzugskanal vorgesehen ist oder nicht, weist die erste Rührstrebe und die zweite Rührstrebe mit Vorteil jeweils mindestens einen Bodenabschnitt auf, wobei sich die Bodenabschnitte im Wesentlichen parallel zur Rührwerk-Drehachse erstrecken und jeweils derart von der Rührwerk-Drehachse beabstandet sind, dass die Bodenabschnitte bei einer Drehung des Rührwerks kurz über dem Behälterboden vorbei streichen. Dadurch ist gewährleistet, dass das Rührwerk-Volumen in Richtung des Behälterbodens betrachtet maximal ausgedehnt ist. Das Merkmal "kurz über dem Behälterboden" ist so auszulegen, dass der Abstand der Bodenabschnitte zum Behälterboden im Bereich weniger Zentimeter liegt.

Es ist dabei vorgesehen, dass die erste Rührstrebe und die zweite Rührstrebe, entlang der Rührwerk-Drehachse betrachtet, relativ zueinander alternierend angeordnete Bodenabschnitte aufweist. Das heißt die erste Rührstrebe und die zweite Rührstrebe weisen entlang ihres Verlaufs keine Bodenabschnitte auf, die spiegelsymmetrisch zur Rührwerk-Drehachse angeordnet sind. Vielmehr ist der Verlauf der ersten Rührstrebe und der zweiten Rührstrebe mit den darin angeordneten Bodenabschnitten derart aufeinander abgestimmt, dass die erste Rührstrebe mit seinen Bodenabschnitten zahnartig in entsprechende Rücksprünge der zweiten Rührstrebe eines im Wesentlichen identisch ausgebildeten Rührwerkes eingreifen kann. Ebenso greifen die Bodenabschnitte der zweiten Rührstrebe in entsprechend ausgebildete Rücksprünge der ersten Rührstrebe ein. Diese Geometrie bringt beim Einsatz einer Mehrzahl derart ausgebildeter und hintereinander angeordneter Rührwerke den Vorteil mit sich, dass sich die Rührwerke auch dann näher beieinander anordnen lassen, wenn benachbarte Rührwerke ohne Phasenverschiebung im gleichen Drehsinn oder im zueinander entgegen gesetzten Drehsinn rotieren. Auf diese Weise überlappen sich die Rührwerk-Volumina benachbarter Rührwerke. Dadurch erfolgt eine intensivere mechanische Durchmischung des biologischen Materials, was zur Homogenisierung der Temperaturverteilung im Fermentationsbehälter und zu einer besseren Gasfreisetzung bei der Produktion von Biogas führt. Das Überlappen der Rührwerk-Volumina reduziert außerdem die Möglichkeit zur Ausbildung von Ablagerungszonen, in denen darin zu liegen kommendes biologisches Material unerwünscht lange im Fermentationsbehälter verbleibt.

Es ist daher vorgesehen, eine Mehrzahl im Wesentlichen gleich ausgebildeter Rührwerke entlang der Behälter-Erstreckungsachse in einem Rührwerk-Drehachsen-Abstand hintereinander anzuordnen, wobei der Rührwerk-Drehachsen-Abstand weniger als das 1,5fache, bevorzugt weniger als das 1,2fache des maximalen Abstandes zwischen der Rührwerk-Drehachse und den Bodenabschnitten der Rührstreben beträgt. Dadurch, dass die Bodenabschnitte der beiden Rührstreben entlang der Rührwerk-Drehachse betrachtet versetzt zueinander angeordnet sind, ist es, wie vorangehend beschrieben, möglich, den Abstand der Rührwerk-Drehachsen zu minimieren. Dabei werden alle benachbarten Rührwerke bevorzugt ohne Phasenverschiebung im gleichen oder im zueinander entgegen gesetzten Drehsinn rotiert.

Eine zweite Variante zum Betreiben der Rührwerke in einer Fermentationsanlage mit einer Mehrzahl im Wesentlichen gleich ausgebildeter Rührwerke sieht vor, dass benachbarte Rührwerke mit gleicher Rotationsgeschwindigkeit gegenläufig zueinander rotiert werden. Wenn dabei eine Phasenverschiebung einer Vierteldrehung zwischen den jeweils benachbarten Rührwerken eingestellt wird, ist eine Kollision der Rührwerke untereinander ausgeschlossen. Bei dieser Variante ist es nicht erforderlich, dass Bodenabschnitte der jeweiligen Rührwerke entlang der Rührwerk-Drehachsen betrachtet alternierend angeordnet sind. Das heißt zum Beispiel, dass beim Erreichen einer senkrechten Stellung eines ersten und dritten Rührwerkes die benachbarten Rührwerke zwei und vier horizontal zu liegen kommen und umgekehrt.

Eine dritte Variante zum Betreiben der Rührwerke in einer Fermentationsanlage mit einer Mehrzahl im Wesentlichen gleich ausgebildeter Rührwerke besteht darin, dass Rührwerk eins und drei senkrecht fixiert stehen und dazwischen nur Rührwerk zwei und vier rotieren. Bei dieser Variante wird von den drehenden Rührwerken zwar weniger Behältervolumen durchgriffen, jedoch kann es möglich sein, dass die Prozessführung bei der Fermentation zumindest zeitweise eine solche Manipulation des biologischen Materials erfordert.

Eine weitere vorteilhafte Variante der Fermentationsanlage sieht vor, dass der Behälterboden einen eben ausgebildeten Mittelbereich mit einer im Wesentlichen rechtwinklig zu den Behälterseitenwänden angeordneten Bodenfläche aufweist. Weiterhin weist der Behälterboden beidseitig benachbart zum Mittelbereich und im stumpfen Winkel vom Mittelbereich zu den Behälterseitenwänden hin ansteigende Flankenbereiche auf. Dies hat den Vorteil, dass über die Flankenbereiche dünnflüssige Abbauprodukte des biologischen Materials in Richtung des Mittelbereiches ablaufen.

Bevorzugt weist der Behälterboden im Mittelbereich einen quer zur Rührwerk-Drehachse orientierten Abzugskanal auf. Dieser Abzugskanal ist üblicherweise mit einem Rost abgedeckt und eignet sich zum Sammeln und Abführen dünnflüssiger Abbauprodukte, die bei der Fermentation beispielsweise in Form organischer Säuren anfallen.

Im Zusammenspiel mit den vorangehend beschriebenen Flankenbereichen ist es vorteilhaft, dass die erste Rührstrebe und die zweite Rührstrebe jeweils mindestens einen Bodenabschnitt aufweisen, wobei die Bodenabschnitte derart von der Rührwerk-Drehachse beabstandet verlaufen, dass die Bodenabschnitte bei einer Drehung des Rührwerks kurz über dem Mittelbereich oder kurz über den Flankenbereichen des Behälterbodens vorbei streichen. Auf diese Weise wird erreicht, dass das Rührwerk-Volumen einen möglichst großen Teil des Behältervolumens erfasst.

Auch im Zusammenhang mit der Ausbildung von schräg abfallenden Flankenbereichen ist es bevorzugt vorgesehen, dass die erste Rührstrebe und die zweite Rührstrebe, entlang der Rührwerk-Drehachse betrachtet, relativ zueinander alternierend angeordnete Bodenabschnitte aufweist. Dadurch wird das bereits beschriebene zahnartige Kämmen zweiter benachbart betriebener Rührwerke ermöglicht. Auf diese Weise lässt sich eine deutliche Überlappung benachbarter Rührwerk-Volumina bei einer hintereinander gereihten Anordnung einer Mehrzahl von Rührwerken erzielen. Diese werden bevorzugt ohne Phasenverschiebung zueinander mit der gleichen Rotationsgeschwindigkeit gleichläufig oder entgegengesetzt zueinander rotiert. Die zu diesem Aspekt vorangehend gemachten Ausführungen gelten entsprechend.

Die Fermentationsantage weist daher eine Mehrzahl von Rührwerken auf, die entlang der Behälter-Erstreckungsachse in einem Rührwerk-Drehachsen-Abstand hintereinander angeordnet sind, der weniger als das 1,5fache, bevorzugt weniger als das 1,2fache des maximalen Abstandes zwischen der Rührwerk-Drehachse und den Bodenabschnitten der Rührstreben beträgt. Auch in diesem Zusammenhang wird auf die vorangehend in Bezug auf die Variante eines im Wesentlichen vollständig ebenen Behälterboden ausgeführten Vorteile verwiesen.

Für sämtliche der vorangehend beschriebenen Fermentationsanlagen ist es von Vorteil, dass mindestens eine Rührstrebe ausgehend vom Umkehrbereich zurück in Richtung der Rührwerk-Drehachse in einem zweiten Befestigungsabschnitt an der Rührwerk-Drehachse festgelegt ist. Dadurch wird insbesondere die beim Rühren des biologischen Materials notwendige Steifigkeit gewährleistet.

Des Weiteren bevorzugt sind der erste Befestigungsabschnitt und der zweite Befestigungsabschnitt an der Rührwerk-Drehachse voneinander beabstandet angeordnet.

Eine für sämtliche vorangehenden Varianten der Fermentationsanlage vorteilhafte Weiterbildung sieht vor, dass eine Mehrzahl voneinander unabhängiger Temperierungseinrichtungen am Fermentationsbehälter und/oder in den Rührwerken vorgesehen ist. Auf diese Weise lässt sich der Fermentationsprozess zusätzlich über eine kontrollierte Wärmezuführung oder Wärmeabführung manipulieren. Dies ist insbesondere für große Volumina in Fermentationsbehältern vorteilhaft. Dazu lassen sich beispielsweise die Rührstreben und die Rührwerk-Drehachse als Hohlprofile ausgestalten und mit einem Medium zum Heizen und/oder Kühlen durchströmen. Die Zuführung des Mediums geschieht bevorzugt über die Rührwerk-Drehachse. Als einfache Ausgestaltung ist der Einsatz von Wasser vorgesehen, das sich aufgrund seiner hohen Wärmekapazität als universelles Medium sowohl für das Heizen als auch für das Kühlen eignet.

Ebenso für alle Varianten der Fermentationsanlage sind die Rührwerke bevorzugt derart ausgebildet, dass die Rührwerk-Drehachse eine Hauptträgheitsachse des Rührwerks bildet. Dadurch ist gewährleistet, dass sich die Rührwerke mit konstantem Kraftaufwand drehen lassen.

Weitere Aspekte und Vorteile der Erfindung werden im Zusammenhang mit der Beschreibung einiger bevorzugter Ausführungsbeispiele in den nachfolgend beschriebenen Figuren verdeutlicht. Die Ausführungsbeispiele sollen den Schutzbereich der Ansprüche jedoch nicht einschränken.

Es zeigt:
- Figur 1: eine schematische Querschnittsansicht des Fermentationsbehälters eines ersten Ausführungsbeispiels der Fermentationsanlage;
- Figur 2: eine schematische Perspektivansicht eines Rührwerks des ersten Ausführungsbeispiels der Fermentationsanlage aus Figur 1;
- Figur 3: eine schematische Perspektivansicht der Fermentationsantage aus Figur 1;
- Figur 4: eine schematische Seitenansicht der Fermentationsanlage aus Figur 1, wobei eine der Behälterseitenwände durchsichtig dargestellt ist;
- Figur 5: eine schematische Aufsicht auf die Fermentationsanlage aus Figur 1;
- Figur 6: eine schematische Querschnittsansicht des Fermentationsbehälters eines zweiten Ausführungsbeispiels der Fermentationsanlage;
- Figur 7: eine schematische Perspektivansicht der zweiten Ausführungsform der Fermentationsanlage aus Figur 6;
- Figur 8: eine schematische Aufsicht auf die Fermentationsanlage aus Figur 6;
- Figur 9: eine schematische Seitenansicht der Fermentationsanlage aus Figur 6, wobei eine der Behälterseitenwände durchsichtig dargestellt ist;

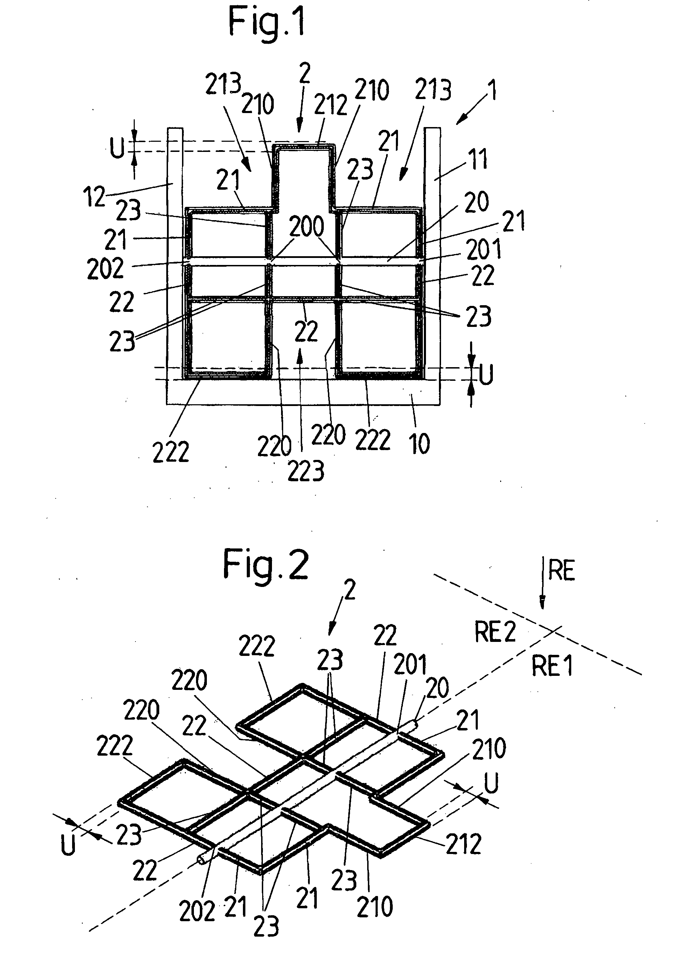

Figur 1 zeigt die schematische Querschnittsdarstellung eines ersten Ausführungsbeispiels einer Fermentationsanlage, bei der in einem Fermentationsbehälter 1 befindliches biologisches Material mittels Rührwerken 2 umgerührt wird.

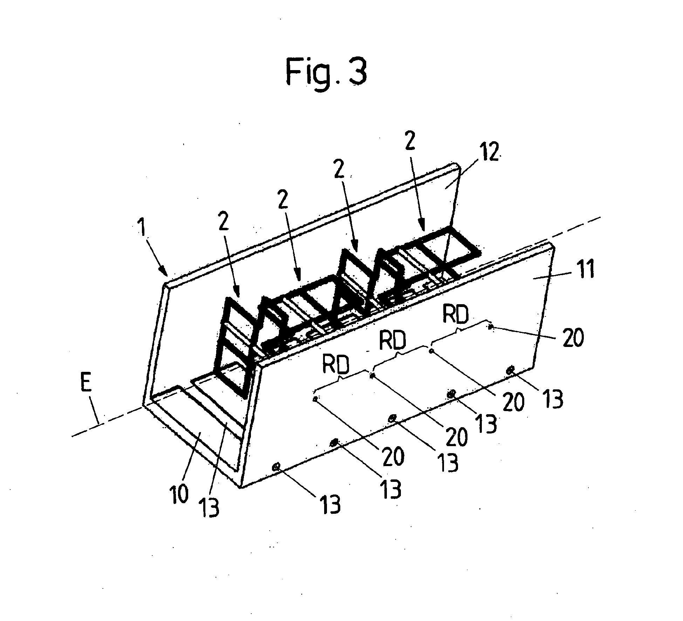

Wie nachfolgend noch ausführlicher in Zusammenhang mit der perspektivischen Darstellung der Fermentationsanlage aus Figur 1 in Figur 3 erläutert, ist der Fermentationsbehälter 1 entlang einer Erstreckungsachse E erstreckt quaderförmig aufgebaut und weist das Querschnittsprofil eines Rechteckes auf. Ein eben ausgebildeter Behälterboden 10 ist von zwei senkrecht zum Behälterboden 10 angeordneten Behälterseitenwänden 11,12 begrenzt. Die Behälterseitenwände 11,12 verlaufen im Wesentlichen parallel zueinander.

Üblicherweise ist ein solcher Fermentationsbehälter 1 mit einer hier nicht dargestellten Abdeckung versehen, über die, beispielsweise bei der Biogasproduktion, das entstehende Biogas gesammelt und abgeleitet wird. Eine derartige Form des Fermentationsbehälters 1 lässt sich auf einfache und kostengünstige Weise z.B. aus Stahlbeton aufbauen. Dadurch ist eine hinreichende Stabilität der Behälterseitenwände 11,12 gegenüber dem Druck des im Fermentationsbehälter befindlichen biologischen Materials gewährleistet. Der Fermentationsbehälter 1 ist im Betrieb regelmäßig fast vollständig mit biologischem Material gefüllt. Füllvolumina derartiger Anlagen belaufen sich oftmals auf viele Dutzend Kubikmeter.

Das Rührwerk 2 ist um eine quer zur Erstreckungsrichtung E des Fermentationsbehälters 1 angeordnete Rührwerk-Drehachse 20 rotierbar. Die Rührwerk-Drehachse 20 ist dazu in hier nicht näher dargestellten Lagerbereichen in den einander gegenüber liegenden Behälterseitenwänden 11,12 drehbar gelagert. Diese Lagerbereiche sind üblicherweise gegenüber dem im Innern des Fermentationsbehälters 1 befindlichen biologischen Material abgedichtet und ermöglichen das Durchtreten der Rührwerk-Drehachse 20 durch eine der Behälterseitenwände 11,12. Auf diese Weise ist es möglich, die Rührwerke 2 einzeln von außen mittels Getriebemotoren anzutreiben. Dadurch sind die Motoren im Wartungs- oder Reparaturfall frei zugänglich.

Das Rührwerk des ersten Ausführungsbeispiels umfasst eine erste Rührstrebe 21 und eine zweite Rührstrebe 22. Beide Rührstreben 21,22 verlaufen ausgehend von einem ersten Drehachsen-Befestigungsabschnitt 201 der Rührwerk-Drehachse 20 in entgegen gesetzte Richtungen zunächst senkrecht von der Rührwerk-Drehachse 20 weg. Der erste Drehachsen-Befestigungsabschnitt 21,22 befindet sich kurz neben der ersten Behälterseitenwand 11. Folglich verlaufen beide Rührstreben 21,22 zunächst parallel zur Ebene der Behälterseitenwand 11 und knicken dann nach unterschiedlich langen Abschnitten rechtwinklig parallel zur Rührwerk-Drehachse 20 orientiert ab. Die erste Rührstrebe 21, die zweite Rührstrebe 22 und die Rührwerk-Drehachse 20 liegen im Wesentlichen in einer gemeinsamen Rührwerk-Ebene RE, wie es insbesondere in der perspektivischen Darstellung des Rührwerks 2 aus Figur 1 in Figur 2 dargestellt ist. Die Rührwerk-Ebene RE ist durch die Rührachse 20 in eine erste Halbebene RE1 und in eine zweite Halbebene RE2 geteilt. Die erste Rührstrebe 21 verläuft dabei vom ersten Drehachsen-Befestigungsabschnitt 201 ausschließlich in der ersten Halbebene RE1 zum zweiten Drehachsen-Befestigungsabschnitt 202. Die zweite Rührstrebe 22 verläuft in der zweiten Halbebene RE2 vom ersten Drehachsen-Befestigungsabschnitt 201 zum zweiten Drehachsen-Befestigungsabschnitt 202. Beim Rührwerk 2 des ersten Ausführungsbeispiels ist der Verlauf der ersten und zweiten Rührstrebe 21,22 jedoch nicht achsensymmetrisch zur Rührwerk-Drehachse 20 ausgebildet.

Die zweite Rührstrebe 22 verläuft ausgehend von der Rührwerk-Drehachse 20 senkrecht zur Rührwerk-Drehachse 20 orientiert bis in einen so genannten Umkehrbereich U. Dieser Umkehrbereich U der zweiten Rührstrebe befindet sich im Wesentlichen in gleichem Abstand zur Rührwerk-Drehachse 20, wie der Behälterboden 10 von der im Wesentlichen parallel zum Behälterboden 10 orientierten Rührwerk-Drehachse 20. Im Umkehrbereich U knickt die zweite Rührstrebe 22 im Wesentlichen rechtwinklig in Richtung der Behältermitte ab und verläuft etwas mehr als ein Drittel der Länge der Rührwerk-Drehachse 20 als ein so genannter Bodenabschnitt 222 der zweiten Rührstrebe 22. Dieser Bodenabschnitt 222 streicht bei der Rotation des Rührwerkes 2 kurz über dem Behälterboden vorbei. Der Abstand zwischen der zweiten Rührstrebe 22 und dem Behälterboden 11 beträgt dabei nur wenige Zentimeter. Von dem zur Behältermitte hin orientierten Endabschnitt des Bodenabschnittes 222 knickt die zweite Rührstrebe 22 in Richtung der Rührwerk-Drehachse 20 ab und läuft auf diese zu. Bevor die zweite Rührstrebe 22 die Rührwerk-Drehachse 20 erreicht, ist ein weiterer Knick der zweiten Rührstrebe 22 vorgesehen. Die zweite Rührstrebe verläuft dann wieder parallel zur Rührwerk-Drehachse 20 über die Behältermitte hinaus. Im Verlauf erfolgt ein weiterer Knick in radialer Richtung, um wieder im Umkehrbereich U einen Bodenbereich 222 auszubilden. Dieser zweite Bodenbereich 222 der zweiten Rührstrebe 22 erstreckt sich dann bis kurz vor die Behälterseitenwand 12, um nach einem erneuten Knick der zweiten Rührstrebe 22 parallel zur Behälterseitenwand 12 wieder zurück zur

Rührachse 20 zu verlaufen. An der Rührachse 20 ist die zweite Rührstrebe 22 in einem zweiten Drehachsen-Befestigungsabschnitt 202 mechanisch an der Rührwerk-Drehachse 20 festgelegt. Zwischen den beiden Bodenbereichen 222 bildet die zweite Rührstrebe in Ihrem Verlauf einen einseitig geöffneten Rücksprungbereich 223 aus. Der Verlauf der ersten Rührstrebe 21 innerhalb der ersten Halbebene RE1 der Rührwerkebene RE ist zwischen dem ersten und dem zweiten Drehachsen-Befestigungsbereich 201,202 derart ausgebildet, dass nur ein einziger Bodenabschnitt 212 vorgesehen ist. Dieser Bodenabschnitt 212 der ersten Rührstrebe 21 verläuft parallel zur Rührwerk-Drehachse 20 und ist entsprechend den Bodenabschnitten 222 der zweiten Rührstrebe 22 zur Rührwerk-Drehachse 20 beabstandet. Die Breite des Bodenabschnittes 212 ist derart gewählt, dass dieser Bodenabschnitt 212 eine etwas geringere Ausdehnung aufweist, als die beiden Bodenabschnitte 222 der zweiten Rührstrebe 22 durch den Rücksprungbereich 223 voneinander beabstandet sind. Beidseitig symmetrisch benachbart ist dieser Bodenabschnitt 212 durch zwei Rücksprungbereiche 213. Dadurch ist gewährleistet, dass die jeweils maximal von der Rührwerk-Drehachse 20 beabstandeten Bodenabschnitte 212,222 der ersten und zweiten Rührstrebe 21,22 bei einer vollen Umdrehung des Rührwerkes 2 zusammengesetzt betrachtet im Wesentlichen die Mantelfläche eines Zylinders beschreiben, dessen Radius dem Abstand von Rührwerk-Drehachse 20 zu den Bodenabschnitten 212,222 entspricht. Dieser fiktive Zylinder stellt das so genannte Rührwerkvolumen dar. Die Rührwerke 2 der Fermentationsanlage haben keine Aufgabe zum Transportieren biologischen Materials. Sie dienen im Wesentlichen einer möglichst homogenen Durchmischung des fermentierenden biologischen Materials. Dabei bestimmen die Eigenschaften des Fermentations-Substrates die konkrete Ausgestaltung des Rührwerkes 2. Wichtig für eine gute Durchmischung ist, dass Rührwerke Rührstrebenabschnitte aufweisen, die sich bei der Rotation des Rührwerkes 2 im Innern des Rührwerk-Volumens bewegen. Diese so genannten Rührstreben-Volumenabschnitte 210,220 sind sowohl im Verlauf der ersten Rührstrebe 21 als auch im Verlauf der zweiten Rührstrebe 22 flankierend zu den jeweiligen Bodenabschnitten 212,222 angeordnet. Durch die Drehbewegung der Rührwerke 2 wird außerdem das Aufsteigen von Gasbläschen unterstützt, die bei der Fermentation entstehen.

Zusätzlich kann es von Vorteil sein so genannte Rührzusatzstreben 23 vorzusehen. Diese Rührzusatzstreben 23 verlaufen zwischen zwei Rührstreben-Kontaktabschnitten einer Rührstrebe oder zwischen einem Rührstreben-Kontaktabschnitt und einem Drehachsen-Kontaktabschnitt derart, dass die Rührzusatzstreben 23 bei der Rotation des Rührwerks 2 innerhalb des Rührwerk-Volumens umlaufen und somit die Durchmischung im Fermentationsbehälter 1 optimieren. Die Anzahl und Positionierung von Rührzusatzstreben 23 kann an die Anforderungen unterschiedlicher Fermentations-Substrate angepasst werden. Ebenso ist denkbar, Befestigungsmittel derart vorzusehen, die ein nachträgliches Hinzufügen oder Entfernen von Rührzusatzstreben 23 erlauben. Auf diese Weise ist die gesamte Fermentationsantage im gewissen Rahmen flexibel an unterschiedliches biologisches Material anpassbar.

Die bereits erwähnte Figur 3 zeigt eine perspektivische Ansicht des ersten Ausführungsbeispiels der Fermentationsanlage gemäß Figur 1. Es ist erkennbar, dass im quaderförmigen Fermentationsbehälter 1 entlang seiner Erstreckungsachse E vier im Wesentlichen gleiche Rührwerke 2 äquidistant zueinander angeordnet sind. Aus Gründen der besseren Übersichtlichkeit sind in dieser Darstellung keine Vorrichtungen gezeigt, mit denen das Zuführen biologischen Materials an einem Ende des Behälters und das Entnehmen des fermentierten biologischen Materials am anderen Ende des Fermentationsbehälters 1 realisiert wird.

Außerdem ist erkennbar, dass im Behälterboden 10 Abzugskanäle 13 eingelassen sind, die sich quer zur Erstreckungsachse E des Fermentationsbehälters 1 erstrecken. Diese Abzugskanäle 13 dienen dem Sammeln und Abführen dünnflüssiger Abbauprodukte, die bei der Fermentation des biologischen Materials im Fermentationsbehälter 1 entstehen.

Der Abstand RD der Rührachsen 20 kann so gewählt werden, dass eine gleich schnelle Rotation aller Rührwerke 2 ohne Phasenverschiebung kollisionsfrei möglich ist. Wenn die Rührwerke 2 ohne Phasenverschiebung in gleicher Drehrichtung oder mit gegenläufigen Drehrichtungen rotieren, so kommen alle Rührwerke 2 zu einem definierten Zeitpunkt mit Ihren Rührwerk-Ebenen RE gemeinsam horizontal zu liegen. In dieser Position greift der Bodenabschnitt 212 mit den beiden flankierenden Rührstreben-Volumenabschnitten 210 der ersten Rührstrebe 21 eines ersten Rührwerkes 2 in den Rücksprungbereich 223 der zweiten Rührstrebe eines benachbart zu liegen kommenden Rührwerkes 2 ein. Umgekehrt umschließen die beiden Bodenabschnitte 222 einer zweiten Rührstrebe 22 den Bodenabschnitt 212 einer ersten Rührstrebe 21, wobei die beiden benachbarten Rücksprungbereiche 213 abgedeckt werden. Ähnlich einem Puzzle ergänzen sich die Rührwerke 2 in dieser horizontalen Lage derart, dass diese mit ihren Rührstreben 21,22 eine dem Behälterboden 10 entsprechende Fläche vollständig umschreiben. Dadurch ist es möglich, den Rührwerk-Drehachsenabstand RD zu reduzieren. Auf diese Weise wird der Überlapp benachbarter Rührwerk-Volumina vergrößert, was wiederum eine verbesserte Durchmischung zur Folge hat und auch Ablagerungsbereiche für biologisches Material oder deren Abbauprodukte verhindert. Ein weiterer Vorteil besteht darin, dass es unabhängig von der jeweiligen Position eines Rührwerkes 2 möglich ist, ein Rührwerk zu fixieren und nur das benachbarte Rührwerk zu rotieren. Dadurch sind mehr Freiheitsgrade bei der Steuerung der gewünschten Durchmischung gegeben.

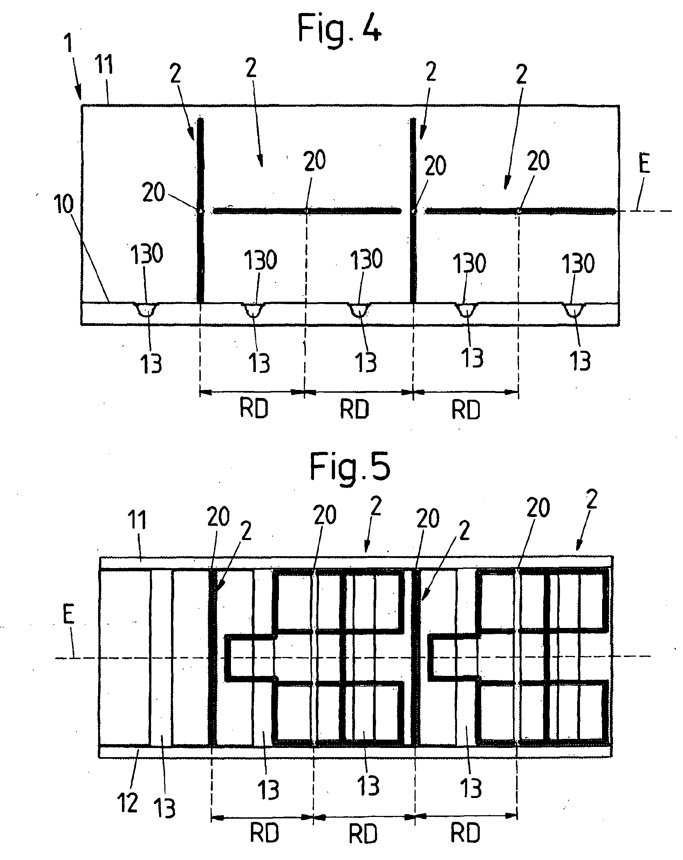

Ebenso ist es denkbar, die Rührwerke 2, wie in Figur 4 angedeutet, phasenverschoben zu betreiben. Figur 4 zeigt die in Figur 3 dargestellte erste Ausführungsform der Fermentationsanlage in einer Seitenansicht, wobei die erste Behälterwand transparent angelegt ist, um die Rührwerke 2 erkennen zu können. Man erkennt von der linken Seite her betrachtet, dass die Rührwerke jeweils um 90°, d.h. eine Vierteldrehung, phasenverschoben zueinander angeordnet sind. Wenn in dieser Konstellation alle Rührwerke mit der gleichen Rotationsgeschwindigkeit und benachbart mit gegenläufigem Drehsinn betrieben werden, so kommt es zu keinem Zeitpunkt zu dem vorangehend beschriebenen puzzle-artigen Ineinandergreifen benachbarter Rührwerke 2. Der Rührwerk-Drehachsenabstand RD kann daher noch weiter reduziert werden, als beim vorangehenden Szenario einer Rotation ohne Phasenverschiebung.

Bei einem Betriebe mit der Phasenverschiebung einer Vierteldrehung gemäß der Darstellung in den Figuren 3 bis 5 ist es grundsätzlich auch denkbar, Rührwerke einzusetzen, die bei der Rotation ohne Phasenverschiebung mit gleicher Rotationsgeschwindigkeit aufgrund der Rührstrebengeometrie miteinander kollidieren würden. Ebenso ist denkbar, die horizontal stehenden Rührwerke 2 zu fixieren und nur die dazwischen liegenden Rührwerke 2 zu rotieren.

Aufgrund der puzzle-artigen Geometrie bietet sich die Möglichkeit, die Rührwerke 2 in einem Betriebsbereich mit einer Phasenverschiebung größer null und kleiner einer Vierteldrehung zu betreiben. Je nach dem, ob die benachbarten Rührwerke 2 im gleichen oder im entgegen gesetzten Drehsinn oder in beiden Betriebszuständen einsetzbar sein sollen, ist der Rührwerk-Drehachsenabstand RD entsprechend anzupassen.

Weiterhin ist in Figur 4 zu erkennen, dass die ebenfalls äquidistant vorgesehenen Abzugskanäle 13 zum Innern des Fermentationsbehälters 1 hin mit einem Abdeckrost 130 abgedeckt sind. Dieser Abdeckrost 130 ermöglicht in Abhängigkeit von seiner Öffnungsgröße das Durchtreten dünnflüssiger Abbauprodukte und verhindert gleichzeitig das Verstopfen der Abzugskanäle 13 durch grobe Bestandteile des biologischen Materials. Die optimierte Durchmischung des biologischen Materials mittels der vorangehend beschriebenen Ausgestaltung der Rührwerke 2 und der vergrößerte Überlapp der benachbarten Rührwerk-Volumina trägt außerdem dazu bei, dass insbesondere in der Nähe der Abzugskanäle 13 keine Ablagerungsbereiche entstehen. Derartige Ablagerungsbereiche könnten mit in diesen Bereichen stagnierendem biologischem Material die Funktion der Abzugskanäle 13 beeinträchtigen.

Figur 5 zeigt die Aufsicht auf die in Figur 4 gezeigte Situation der Fermentationsanlage. Man erkennt, dass eine gleichzeitige horizontale Stellung aller vier Rührwerke 2 trotz der Puzzle-Geometrie ineinander greifender Abschnitte nicht möglich wäre. Dafür müsste der Rührwerk-Drehachsenabstand RD vergrößert werden. Jedoch ist es aufgrund dieser Geometrie bis zu einer gewissen Grenzbereich möglich, die Phasenverschiebung zwischen den Rührwerken 2 bei der Rotation von der dargestellten Vierteldrehung zu reduzieren.

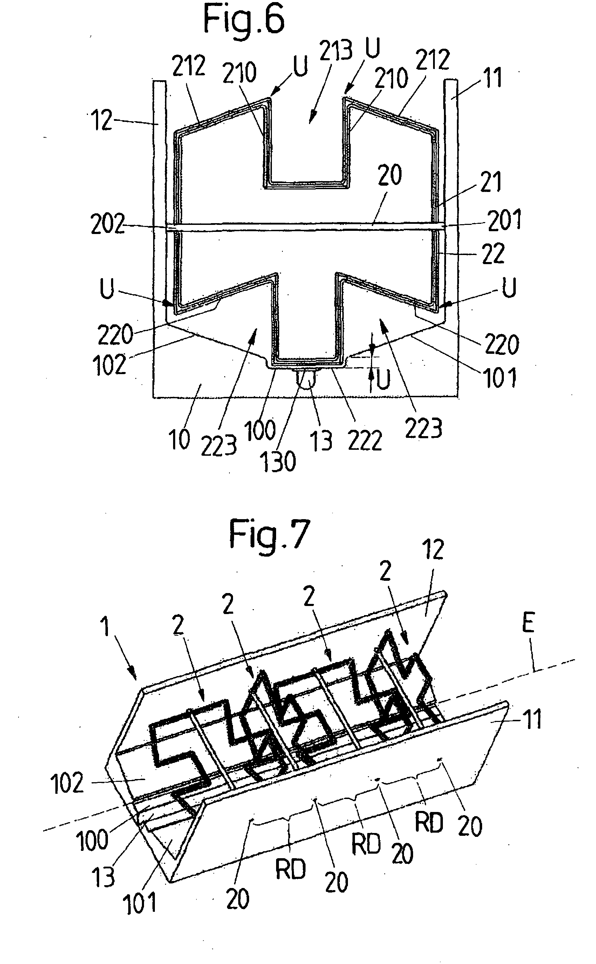

Die Figuren 7 bis 9 zeigen entsprechend den Darstellungen der Figuren 1, 3 bis 5 ein zweites Ausführungsbeispiel einer Fermentationsanlage mit ebenen Rührwerken 2.

Gleiche Bauelemente sind mit gleichen Bezugszeichen versehen. Im Umfang übereinstimmender Strukturen und Funktionen gelten vollumfänglich die vorangehend gemachten Ausführungen. Nachfolgend werden daher im Wesentlichen nur die Unterschiede beschrieben.

Anders als beim ersten Ausführungsbeispiel ist der Fermentationsbehälter 1 mit einem Behälterboden 10 ausgebildet, der einen rechtwinklig zu den Behälterseitenwänden 11,12 angeordneten Mittelbereich 100 aufweist. Dies ist insbesondere in Figur 6 dargestellt. Dieser Mittelbereich 100 ist deutlich schmaler als der Abstand der Behälterseitenwände 11,12 und weist parallel zur Drehachse 20 eines Rührwerkes 2 betrachtet eine Ausdehnung auf, die geringfügig breiter ist als der Bodenabschnitt 222 der zweiten Rührstrebe 22. Beidseitig benachbart zum ebenen Mittelbereich 100 fallen im stumpfen Winkel von den Behälterseitenwänden 11,12 betrachtet Flankenbereiche 101,102 von den Behälterseitenwänden 11,12 zum Mittelbereich 100 hin ab. Die erste Rührstrebe 21 weist entsprechend der Ausgestaltung des Behälterbodens 10 zwei Bodenabschnitte 212 derart auf, dass diese bei einer Drehung des Rührwerks 2 kurz über den Flankenbereichen 101,102 vorbei streichen. Zwischen diesen beiden Bodenabschnitten 212 der ersten Rührstrebe 21 ist wiederum ein Rücksprungbereich 213 vorgesehen. Dieser Rücksprungbereich 213 ist derart dimensioniert, dass der Bodenabschnitt 222 der zweiten Rührstrebe 22 zusammen mit den beiden flankierenden Rührstreben-Volumenabschnitten 220 in diesen Rücksprungbereich 213 eingreifen kann. Folglich ergeben sich die gleichen funktionalen Vorteile, die im Zusammenhang mit dem ersten Ausführungsbeispiel bereits beschrieben worden sind.

Die im stumpfen Winkel zum Mittelbereich 100 hin abfallenden Flankenbereiche 101,102 sorgen zusammen mit den kurz darüber vorbei streichenden Bodenabschnitten 212 des Rührwerks 2 dafür, dass dünnflüssige Abbauprodukte der Fermentation von der Schwerkraft unterstützt in Richtung des Mittelbereichs abfließen. Im Mittelbereich ist ein entlang der Erstreckungsachse E des Fermentationsbehälters 1 erstreckter Abzugskanal 13 mit einem Abdeckrost 130 vorgesehen.

Anders als das Rührwerk 2 des ersten Ausführungsbeispiels umfasst das in Figur 6 gezeigte Rührwerk 2 des zweiten Ausführungsbeispiels mehr Umkehrbereiche U. Die zweite Rührstrebe 22 verläuft vom ersten Drehachsen-Befestigungsabschnitt 201 zunächst parallel zur unmittelbar benachbarten Behälterseitenwand 11 des Fermentationsbehälters 1. Dieser Verlauf endet in einem ersten Umkehrbereich U, kurz bevor die zweite Rührstrebe 22 auf den von der Behälterseitenwand 11 im stumpfen Winkel abfallenden Flankenbereich 101 des Behälterbodens 10 stößt. Die zweite Rührstrebe 22 knickt in diesem ersten Umkehrbereich U in einem spitzen Winkel in Richtung des Innern des Fermentationsbehälters 1 ab und nähert sich dabei, unter Bildung eines Rührstreben-Volumenabschnittes 220 wieder der Rührwerk-Drehachse 20. Der spitze Winkel der zweiten Rührstrebe 22 im ersten Umkehrbereich U entspricht im Wesentlichen der Differenz von 180° und dem stumpfen Winkel mit dem der Flankenbereich 101 von der Behälterseitenwand 11 abknickt.

Sobald die zweite Rührstrebe 22 in der senkrechten Projektion auf den Behälterboden 10 betrachtet den Beginn des ebenen Mittelbereiches 100 erreicht, knickt die zweite Rührstrebe 22 im gleichen spitzen Winkel in Richtung des Mittelbereiches 100 ab. Kurz vor dem Erreichen des Mittelbereiches 100 knickt die zweite Rührstrebe 22 um zirka 90° ab, um im Wesentlichen parallel zum Mittelbereich 100 in einem Bodenabschnitt 222 zu verlaufen. Die Rührwerk-Drehachse 20 und der Bodenabschnitt 222 sind dabei derart voneinander beabstandet, dass der Bodenabschnitt 222 bei einer Drehung des Rührwerkes 2 kurz über dem Mittelbereich 100 vorbei streicht.

Der weitere Verlauf der zweiten Rührstrebe 22 vom Bodenabschnitt 222 zum zweiten Drehachsen-Befestigungsabschnitt 202 erfolgt spiegelsymmetrisch zu der in der Rührwerksebene RE (entspricht der Querschnittsebene in Figur 6) verlaufenden Mittelsenkrechten der Rührwerk-Drehachse 20. Dabei bildet der Bodenabschnitt 222 einen weiteren Umkehrbereich U im Sinne der vorliegenden Erfindung.

Die erste Rührstrebe 21 verläuft vom ersten Drehachsen-Befestigungsabschnitt 201 ausgehend zunächst parallel zur benachbarten Behälterseitenwand 11, um dann mit einem solchen stumpfen Winkel in Richtung des Innern des Fermentationsbehälters 1 abzuknicken, mit dem die Flankenbereiche 101,102 jeweils von den Behälterseitenwänden 11,12 ausgehend zum Mittelbereich 100 hin abknicken. Dabei bildet der abgeknickt verlaufende Bereich der ersten Rührstrebe 21 einen Bodenabschnitt 212. Dieser Bodenabschnitt 212 entspricht in seiner Breite der Breite der Flankenbereiche 101,102.

An seinem von der Behälterseitenwand 11 beabstandeten Ende knickt der Bodenabschnitt 212 in einem Umkehrabschnitt U in einem spitzen Winkel in Richtung der Rührwerk-Drehachse ab, wobei der weitere Verlauf der ersten Rührstrebe einen rechteckförmigen Rücksprungbereich 213 bildet. In diesem Rücksprungbereich 213 verläuft die erste Rührstrebe 21 in Rührstreben-Volumenabschnitten 210. Entsprechend dem vorangehend beschriebenen Verlauf der zweiten Rührstrebe 22 ist der weitere Verlauf der ersten Rührstrebe 21 bis zum zweiten Drehachsen-Befestigungsabschnitt 202 achsensymmetrisch zu der in der Rührwerkebene RE liegenden Mittelsenkrechten der Rührwerk-Drehachse 20 ausgebildet.

Das Rührwerk 2 des zweiten Ausführungsbeispiels ist ohne Rührzusatzstreben 23 ausgebildet. Bei diesem Beispiel ist die verbesserte Durchmischung und Gasfreisetzung durch einen Verlauf der Rührstreben 21,22 gewährleistet, der Rührstreben-Volumenabschnitte 210,220 aufweist. Selbstverständlich ist es möglich, in der Darstellung des Rührwerks 2 gemäß Figur 6 Rührzusatzstreben 23 vorzusehen. Diese würden entweder zwischen der Rührwerks-Drehachse 20 und den Rührstreben 21,22 oder zwischen zwei Abschnitten von Rührstreben 21,22 verlaufen.

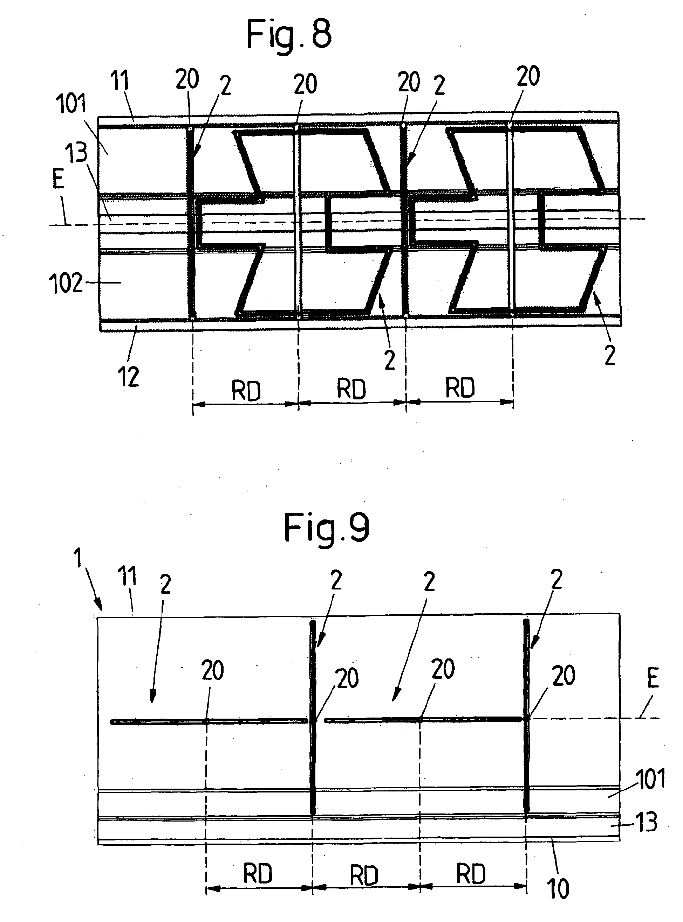

Die im Hinblick auf die Figuren 5 und 6 gemachten Ausführungen gelten entsprechend für die Darstellungen in den Figuren 8 und 9.

Die Ausbildung und die Geometrie der Rührstreben 21,22 und der Rührzusatzstreben 23 ist sowohl für das ebene Rührwerk 2 des ersten Ausführungsbeispiels als auch für das ebene Rührwerk 2 des zweiten Ausführungsbeispiels bevorzugt derart austariert, dass neben der optimierten Durchmischung auch gewährleistet ist, dass die Rührachse 20 durch eine der Hauptträgheitsachsen des Rührwerkes 2 verläuft. Für einen einfachen Aufbau kann es bei den eben ausgebildeten Rührwerken 2 von Vorteil sein, dass die Rührstreben 21,22 und die Rührzusatzstreben 23 in Bezug zu einer in der Rührwerk-Ebene RE verlaufenden Mittelsenkrechten achsensymmetrisch angeordnet sind. Dieses technische Merkmal ist selbstverständlich über die beiden gezeigten Ausführungsbeispiele ebener Rührwerke hinaus für sämtliche Varianten ebener Rührwerke gültig und anwendbar.

Zusammenfassend ist festzustellen, dass die beiden Ausführungsbeispiele der Fermentationsanlage mit ebenen Rührwerken 2 sehr vielseitige Möglichkeiten hinsichtlich unterschiedlicher Ansteuerszenarien der im Fermentationsbehälter 1 benachbarten Rührwerke 2 bieten.

Hier gilt entsprechend den Ausführungen zu den ebenen Rührwerken, dass die Anzahl und Positionierung von Rührzusatzstreben in Abwägung der dadurch erzielten stärkeren Mischwirkung und der zwangsläufig mit verursachten erhöhten Schwergängigkeit beim Rührvorgang erfolgen muss.

Für alle Ausführungsbeispiele gilt gemeinsam, dass der Fermentationsbehälter 1 wärmeisoliert derart ausgebildet ist, dass die Fermentation sowohl mesophil als auch thermophil ablaufen kann. Sowohl der Fermentationsbehälter 1 als auch die Rührwerke 2 können dazu mit nicht näher dargestellten Temperierungseinrichtungen versehen sein.

In einer Variante ist vorgesehen, die Rührwerk-Streben zumindest teilweise mit Hohlprofilen aufzubauen, die die sich von einem Medium, beispielsweise Wasser je nach Bedarf zum Heizen oder zum Kühlen durchströmen lassen. Zur Beheizung kann die Abwärme eines Blockheizkraftwerkes eingesetzt werden. Um im Fermentationsbehälter 1 bei Bedarf Temperaturgradienten einstellen zu können, ist es vorteilhaft, eine Mehrzahl voneinander unabhängiger Temperierungseinrichtungen am Fermentationsbehälter und/oder an den Rührwerken vorzusehen.

Ebenfalls für alle Ausführungsbeispiele und unabhängig davon für alle Rührwerkstypen gilt, dass die Endbereiche des Fermentationsbehälters 1 entlang seiner Erstreckungsachse E betrachtet derart ausgebildet sind, dass dort die Ausbildung von Stagnationszonen, in denen sich Material außerhalb der Reichweite der Rührwerke 2 anlagern kann, vermieden bzw. minimiert wird. Dies lässt sich durch entsprechend schräg bzw. gekrümmt ausgebildete Behälter-Endabschnitte erreichen. Idealerweise ist die Krümmung derart vorgesehen, dass das zum Endabschnitt benachbarte Rührwerk mit seinen Rührstreben knapp über dem gekrümmten Flächen des Endabschnittes gleitet. Es ist selbstverständlich, dass bei der Formgebung der Behälter-Endabschnitte die Strukturen der Vorrichtungen für die Be- und Entnahme von Material berücksichtigt werden muss.

### Bezugszeichenliste:

Fermentationsbehälter 1
   Behälterboden 10
   Mittelbereich 100
   Flankenbereiche 101,102
   gekrümmte Bodenkontur 103
Behälterseitenwände 11,12
   Abzugskanal 13
   Abdeckrost 130
Rührwerk 2
   Rührwerk-Drehachse 20
   erster Drehachsen-Befestigungsabschnitt 201
   zweiter Drehachsen-Befestigungsabschnitt 202
Rührstreben 21,22
   Rührstreben-Volumenabschnitte 210,220
   Bodenabschnitt 212,222
   Rücksprungbereich 213,223
   Rührzusatzstrebe 23
Behälter-Erstreckungsachse E
   Rührwerk-Drehachsen-Abstand RD
   Rührwerk-Ebene RE
   erste Halbebene der Rührwerk-Ebene RE1
   zweite Halbebene der Rührwerk-Ebene RE2
   Umkehrbereich U

## Patentansprüche

1. Fermentationsanlage für biologisches Material mit folgenden Merkmalen:
- ein Fermentationsbehälter (1) zur Aufnahme biologischen Materials mit einem eben ausgebildeten Behälterboden (10), der eine rechtwinklig zu zwei parallel zueinander angeordneten Behälterseitenwänden (11,12) verlaufende geradlinige Bodenkontur aufweist, wobei der Fermentationsbehälter (1) entlang einer Behälter-Erstreckungsachse (E) erstreckt ausgebildet ist, und
- eine Mehrzahl jeweils um eine Rührwerk-Drehachse (20) rotierbar angeordnete gleiche Rührwerke (2) zum Rühren aufgenommenen biologischen Materials, wobei die Rührwerke (2) entlang der Behälter-Erstreckungsachse (E) in einem Rührwerk-Drehachsen-Abstand (RD) hintereinander angeordnet sind, wobei die Rührwerke (2) mindestens eine erste Rührstrebe (21) und eine zweite Rührstrebe (22) aufweisen, die jeweils im Bereich der Rührwerk-Drehachse (20) festgelegt ist, und die Rührwerk-Drehachsen (20) senkrecht zur Behälter-Erstreckungsachse (E) angeordnet sind, wobei der Fermentationsbehälter (1) entlang der Rührwerks-Drehachse (20) in Richtung des Behälterbodens geschnitten eine Querschnittskontur mit den zwei parallel zueinander verlaufenden senkrecht zu den Rührwerks-Drehachsen (20) angeordneten Behälterseitenwänden (11,12) aufweist, zwischen denen sich der Behälterboden (10) mit der den Fermentationsbehälter nach unten angrenzenden geradlinigen Bodenkontur erstreckt,
wobei die erste Rührstrebe (21) und die zweite Rührstrebe (22) jeweils mindestens einen Bodenabschnitt (212,222) aufweisen, wobei sich die Bodenabschnitte (212,222) parallel zu der Rührwerk-Drehachse (20) erstrecken und jeweils derart von der Rührwerk-Drehachse (20) beabstandet sind, dass die Bodenabschnitte (212,222) bei einer Drehung des Rührwerks (2) kurz über dem Behälterboden (10) vorbei streichen,
wobei der Rührwerk-Drehachsen-Abstand (RD) weniger als das 1,5fache, bevorzugt weniger als das 1,2fache des maximalen Abstandes zwischen der Rührwerk-Drehachse (20) und dem Behälterboden (10) beträgt,
**dadurch gekennzeichnet,**
**dass** die erste Rührstrebe (21) und die zweite Rührstrebe (22) jeweils ausgehend von einem ersten Drehachsen-Befestigungsabschnitt (201) im Bereich der Rührwerk-Drehachse (20) über einen von der Rührwerk-Drehachse beabstandeten Umkehrbereich (U) derart zurück in Richtung der Rührwerk-Drehachse (20) verlaufen, dass die Rührstreben (21,22) das im Fermentationsbehälter befindliche biologische Material nicht nur auf dem Weg ausgehend von der Rührwerk-Drehachse (20) hin zum Umkehrbereich (U) sondern zusätzlich auch noch in weiteren Bereichen, die zwischen dem Umkehrbereich (U) und der Rührwerk-Drehachse (20) angeordnet sind, durchgreifen und
**dass** die erste Rührstrebe (21) und die zweite Rührstrebe (22), entlang der Rührwerk-Drehachse (20) betrachtet, relativ zueinander alternierend angeordnete Bodenabschnitte (212,222) aufweisen.

2. Fermentationsanlage gemäß Anspruch 1, **dadurch gekennzeichnet, dass** die Rührwerke (2) bei einer Rotation um die Rührwerk-Drehachsen (20) Rührwerk-Volumen umschreiben und die Rührstreben (21,22) des Rührwerks (2) zumindest abschnittsweise in Form von Rührstreben-Volumenabschnitten (210,220) jeweils im Inneren des Rührwerk-Volumens verlaufen und/oder die Rührwerke (2) im Inneren des jeweils umschriebenen Rührwerk-Volumens mindestens eine sich bei der Rotation des Rührwerks (2) mitdrehende Rührzusatzstrebe (23) aufweisen.

3. Fermentationsanlage gemäß Anspruch 2, **dadurch gekennzeichnet, dass** sich die Rührzusatzstreben (23) zwischen zwei Rührstreben-Kontaktabschnitten oder zwischen einem Rührstreben-Kontaktabschnitt und einem Drehachsen-Kontaktabschnitt im Bereich der jeweiligen Rührwerk-Drehachse (20) erstreckt.

4. Fermentationsanlage gemäß einem der Ansprüche 2 oder 3, **dadurch gekennzeichnet, dass** die Rührstreben (21,22) und die Rührzusatzstrebe (23) in einer Rührwerk-Ebene (RE) angeordnet sind, in der auch die Rührwerk-Drehachse (20) verläuft.

5. Fermentationsanlage gemäß Anspruch 4, **dadurch gekennzeichnet, dass** das Rührwerk (2) die erste Rührstrebe (21) und die zweite Rührstrebe (22) derart aufweist, dass die erste Rührstrebe (21) in einer ersten Halbebene (RE1) der Rührwerk-Ebene (RE) verläuft und die zweite Rührstrebe (22) in einer durch die Rührwerk-Drehachse (20) von der ersten Halbebene (RE1) getrennten zweiten Halbebene (RE2) der Rührwerk-Ebene (RE) verläuft.

6. Fermentationsanlage gemäß einem der vorangehenden Ansprüche, **dadurch gekennzeichnet, dass** mindestens eine der Rührstreben (21,22) ausgehend vom Umkehrbereich (U) zurück in Richtung der Rührwerk-Drehachse (20) in einem zweiten Drehachsen-Befestigungsabschnitt (202) an der jeweiligen Rührwerk-Drehachse (20) festgelegt ist.

7. Fermentationsanlage gemäß Anspruch 6, **dadurch gekennzeichnet, dass** der erste Drehachsen-Befestigungsabschnitt (201) und der zweite Drehachsen-Befestigungsabschnitt (202) voneinander beabstandet angeordnet sind.

8. Fermentationsanlage gemäß einem der vorangehenden Ansprüche, **dadurch gekennzeichnet, dass** die Rührwerke (2) derart ausgebildet sind, dass die Rührwerk-Drehachse (20) jeweils eine Hauptträgheitsachse des Rührwerks (2) bildet.

## Claims

1. A fermentation plant for biological material having the following characteristics:
- a fermentation container (1) for receiving biological material having an evenly formed container bottom (10) having a linear bottom contour running rectangular to two container side walls (11, 12) being parallel to each other, wherein the fermentation container (1) is formed in an extended manner along a container extension axis (E),
- a plurality of same agitators (2) respectively arranged around an agitator rotation axis (20) in a rotatable manner and intended for agitating received biological material, wherein the agitators (2) are arranged consecutively along the container extension axis (E) in an agitator-rotation-distance (RD), wherein the agitators (2) have at least one first agitator bar (21) and a second agitator bar (22) respectively fixed in the region of the agitator rotation axis (20), and the agitator rotation axes (20) are arranged vertically to the container extension axis (E), wherein the fermentation container (1) has along the agitator rotation axis (20) cut in the direction of the container bottom a cross-sectional contour with the two container side walls (11, 12) running parallel to each other and being arranged vertically to the agitator rotation axes (20) and between which the container bottom (10) extends with the straight bottom contour adjacent downwards the fermentation container,
- wherein the first agitator bar (21) and the second agitator bar (22) each have at least a bottom section (212, 222), wherein the bottom sections (212, 222) extend parallel to the agitator rotation axis (20) and each are spaced apart from the agitator rotation axis (20) such that the bottom sections (212, 222) upon rotation of the agitator (2) sweep past just above the container bottom (10), wherein the agitator-rotation axis-distance (RD) is less than 1.5 times, preferably less than 1.2 times the maximum distance between the agitator rotation axis (20) and the container bottom (10),
**characterized in that**
the first agitator bar (21) and the second agitator bar (22) each starting from a first rotation axis mounting section (201) in the region of the agitator rotation axis (20) through a reversal region (U) spaced apart from the agitator rotation axis run back in the direction of agitator rotation axis (20) such that the agitator bars (21, 22) grasp through the biological material being in the fermentation container not only on the way starting from the agitator rotation axis (20) to the reversal region (U) but additionally also in further regions arranged between the reversal region (U) and the agitator rotation axis (20) and
that the first agitator bar (21) and the second agitator bar (22) have seen along the agitator rotation axis (20) bottom sections (212, 222) arranged in an alternating manner relative to each other.

2. The fermentation plant according to claim 1, **characterized in that** the agitators (2) circumscribe upon rotation around the agitator rotation axes (20) an agitator volume and the agitator bars (21, 22) of the agitator (2) each run at least in sections in the form of agitator bar volume sections (210, 220) inside the agitator volume and/or the agitators (2) have in the interior of the respective circumscribed agitator volume at least one additional agitator bar (23) co-rotating upon rotation of the agitator (2).

3. The fermentation plant according to claim 2, **characterized in that** the additional agitator bars (23) extend between two agitator bar contact sections or between an agitator bar contact section and a rotation axis contact section in the region of the respective agitator rotation axis (20).

4. The fermentation plant according to one of claims 2 or 3, **characterized in that** the agitator bars (21, 22) and the additional agitator bar (23) are arranged in an agitator plane (RE), in which the agitator rotation axis (20) is also running.

5. The fermentation plant according to claim 4, **characterized in that** the agitator (2) has the first agitator bar (21) and the second agitator bar (22) such that the first agitator bar (21) runs in a first half-plane (RE1) of the agitator plane (RE) and the second agitator bar (22) runs in a second half-plane (RE2) of the agitator plane (RE) separated from the first half-plane (RE1) by the agitator rotation axis (20).

6. The fermentation plant according to one of the preceding claims, **characterized in that** at least one of the agitator bars (21, 22) is fixed starting from the reversal region (U) back in the direction of agitator rotation axis (20) in a second rotation axis mounting section (202) on the respective agitator rotation axis (20).

7. The fermentation plant according to claim 6, **characterized in that** the first rotation axis mounting section (201) and the second rotation axis mounting section (202) are spaced apart.

8. The fermentation plant according to one of the preceding claims, **characterized in that** the agitators (2) are formed such that the agitator rotation axis (20) forms a main axis of inertia of the agitator (2) respectively.

## Revendications

1. Une installation de fermentation pour une matière biologique ayant les caractéristiques suivantes:
- un réservoir de fermentation (1) destiné à recevoir une matière biologique et ayant un fond de réservoir (10) de forme plane qui a un contour du fond rectiligne et passant à angle droit à deux parois latérales du réservoir (11, 12) disposées en parallèle l'une à l'autre, le réservoir de fermentation (1) étant formé à façon de s'étendre le long de l'axe d'extension du réservoir (E), et
- une pluralité de même agitateurs (2) disposés respectivement de manière rotative autour un axe de rotation de l'agitateur (20) et destinés à agiter la matière biologique reçue, dans laquelle les agitateurs (2) sont disposés le long de l'axe d'extension du réservoir (E) à une distance de l'axe de rotation vers l'agitateur (RD) à la file, dans laquelle les agitateurs (2) ont au moins un premier étai de l'agitateur (21) et un second étai de l'agitateur (22) fixés respectivement dans la région de l'axe de rotation de l'agitateur (20) et les axes de rotation de l'agitateur (20) sont disposés perpendiculaires à l'axe d'extension du réservoir (E), le réservoir de fermentation (1) ayant le long de l'axe de rotation de l'agitateur (20) coupé en direction du fond de réservoir un contour de section transversale avec les deux parois latérales du réservoir (11, 12) parallèles entre eux et disposées perpendiculaires aux axes de rotation de l'agitateur (20) et entre lesquelles le fond de réservoir (10) s'étend avec le contour du fond rectiligne adjacent vers le bas au réservoir de fermentation, dans laquelle le premier étai de l'agitateur (21) et le second étai de l'agitateur (22) ont respectivement au moins une section de fond (212, 222), dans laquelle les sections de fond (212, 222) s'étendent parallèlement à l'axe de rotation de l'agitateur (20) et sont espacées respectivement de l'axe de rotation de l'agitateur (20) de telle manière que les sections de fond (212, 222) passent à côté juste au dessus du fond de réservoir (10) lors de rotation de l'agitateur (2), dans laquelle la distance de l'axe de rotation vers l'agitateur (RD) est moins de 1,5 fois, de préférence moins de 1,2 fois la distance maximale entre l'axe de rotation de l'agitateur (20) et le fond de réservoir (10),
**caractérisée en ce**
**que** le premier étai de l'agitateur (21) et le second étai de l'agitateur (22) passent respectivement partant d'une première section de fixation de l'axe de rotation (201) dans la région de l'axe de rotation de l'agitateur (20) à travers une région d'inversion (U) espacée de l'axe de rotation de l'agitateur en retour vers la direction de l'axe de rotation de l'agitateur (20) de telle manière que les étais de l'agitateur (21, 22) saisissent par la matière biologique étant dans le réservoir de fermentation non seulement sur la voie partant de l'axe de rotation de l'agitateur (20) vers la région d'inversion (U) mais aussi additionnellement dans d'autres régions disposées entre la région d'inversion (U) et l'axe de rotation de l'agitateur (20) et
**que** le premier étai de l'agitateur (21) et le second étai de l'agitateur (22) ont vus le long de l'axe de rotation de l'agitateur (20) des sections du fond (212, 222) disposées de manière alternante relativement les unes aux autres.

2. L'installation de fermentation selon la revendication 1, **caractérisé en ce que** les agitateurs (2) circonscrivent lors d'une rotation autour de l'axe de rotation de l'agitateur (20) un volume d'agitateur et les étais de l'agitateur (21, 22) de l'agitateur (2) passent respectivement au moins par passages sous la forme des sections de volume d'étais de l'agitateur (210, 220) à l'intérieur du volume de l'agitateur et/ou les agitateurs (2) ont à l'intérieur du volume de l'agitateur circonscrite respectivement au moins un étai de l'agitateur additionnel (23) en co-rotation lors de la rotation de l'agitateur (2).

3. L'installation de fermentation selon la revendication 2, **caractérisé en ce que** l'étai de l'agitateur additionnel (23) s'étend entre deux sections de contact des étais de l'agitateur ou entre une section de contact des étais de l'agitateur et une section de contact de l'axe de rotation dans la région de l'axe de rotation de l'agitateur (20) respectif.

4. L'installation de fermentation selon l'une des revendications 2 ou 3, **caractérisé en ce que** les étais de l'agitateur (21, 22) et l'étai de l'agitation additionnel (23) sont disposés dans un plan de l'agitateur (RE), par lequel l'axe de rotation de l'agitateur (20) passe aussi.

5. L'installation de fermentation selon la revendication 4, **caractérisé en ce que** l'agitateur (2) a le premier étai de l'agitation (21) et le second étai de l'agitation (22) de telle manière que le premier étai de l'agitation (21) passe par un premier demi-plan (RE1) du plan de l'agitateur (RE) et le second étai de l'agitation (22) passe par un second demi-plan (RE2) du plan de l'agitateur (RE) séparé du premier demi-plan (RE1) par l'axe de rotation de l'agitateur (20).

6. L'installation de fermentation selon l'une quelconque des revendications précédentes, **caractérisé en ce qu'**au moins un étai de l'agitation (21, 22) est fixé partant de la région d'inversion (U) en retour dans la direction de l'axe de rotation de l'agitateur (20) dans une seconde section de fixation de l'axe de l'agitateur (202) sur l'axe de rotation de l'agitateur respectif (20).

7. L'installation de fermentation selon la revendication 6, **caractérisé en ce que** la première section de fixation de l'axe de l'agitateur (201) et la seconde section de fixation de l'axe de l'agitateur (202) sont disposées à l'écart l'une à l'autre.

8. L'installation de fermentation selon l'une quelconque des revendications précédentes, **caractérisé en ce que** les agitateurs (2) sont formés de telle manière que l'axe de rotation de l'agitateur (20) forme respectivement un axe principal d'inertie de l'agitateur (2).
